(19) Europäisches Patentamt
European Patent Office
Office européen des brevets

(11) Veröffentlichungsnummer: **0 163 320**
**B1**

# (12) EUROPÄISCHE PATENTSCHRIFT

(45) Veröffentlichungstag der Patentschrift: **30.03.88**

(51) Int. Cl.⁴: **C 07 C 143/04**, C 07 C 145/00, C 07 C 139/12

(21) Anmeldenummer: **85106738.9**

(22) Anmeldetag: **31.05.85**

(54) **Mehrbasige Propansulfonsäuren und deren Salze, Verfahren zu ihrer Herstellung und ihre Verwendung.**

(30) Priorität: **01.06.84 DD 263724**
**01.06.84 DD 263719**

(43) Veröffentlichungstag der Anmeldung:
**04.12.85 Patentblatt 85/49**

(45) Bekanntmachung des Hinweises auf die Patenterteilung:
**30.03.88 Patentblatt 88/13**

(84) Benannte Vertragsstaaten:
**AT BE CH DE FR GB IT LI NL SE**

(56) Entgegenhaltungen:
**DD - B - 154 443**
**DE - A - 1 418 746**
**DE - A - 2 313 539**
**DE - C - 1 117 565**

**Chemical Abstracts, Band 52, Nr. 5, 10. März 1958, Columbus, Ohio, USA, W.D. ROLL et al. "Preparation of metal salts of alkanesulfonic acids", Spalte 3663g**

(73) Patentinhaber: **Akademie der Wissenschaften der DDR, Otto-Nuschke-Strasse 22/23, DDR-1086 Berlin (DD)**

(72) Erfinder: **Ballschuh, Detlef, Dr. Dipl.-Chem., Graudenzer Strasse 17, DDR-1034 Berlin (DD)**
Erfinder: **Seibt, Horst, Dr. Dipl.-Chem., Eugen-Schönhaar-Strasse 15, DD-1055 Berlin (DD)**
Erfinder: **Rusche, Jochen, Dr. Dipl.-Chem., Hans-Loch-Strasse 263, DDR-1136 Berlin (DD)**
Erfinder: **Ohme, Roland, Dr. Dipl.-Chem., Waldstrasse 6, DDR-1180 Berlin (DD)**

(74) Vertreter: **Patentanwälte Beetz sen. - Beetz jun. Timpe - Siegfried - Schmitt-Fumian, Steinsdorfstrasse 10, D-8000 München 22 (DE)**

EP 0 163 320 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft mehrbasige Sulfonsäuren des Propans und 2-Methylpropans und deren Salze, Verfahren zu ihrer Herstellung sowie ihre Verwendung als Hydrotropika.

Es wurde gefunden, dass diese neuen Verbindungen als Zusatzstoffe zur Herstellung von bestimmten hydrotropen wässrigen Systemen industriell, insbesondere in der chemischen Industrie, verwendet werden können. Des weiteren sind einige dieser Verbindungen auch als reaktive Zwischenprodukte einsetzbar. Ferner dienen Propantrisulfonsäure und ihre Salze zur Gewinnung anderer löslicher Metallsalze, die beispielsweise in galvanischen Elektrolyten verwendbar sind.

Mehrbasige Propansulfonsäuren wurden bisher noch nicht beschrieben.

Aus der DE-AS 1418746 und der DE-OS 2313539 ist eine radikalische Difunktionalisierung mit Hydrogensulfiten bei Olefinen und ungesättigten Alkoholen bekannt, die unter dem Einfluss von organischen Radikalbildnern oder Luftsauerstoff im pH-Wertbereich von 4 bis 9 und vorzugsweise 4 bis 5 verläuft. Hierbei sind in der Regel Reaktionszeiten von einer bis zu mehreren Stunden erforderlich. Auf diese Weise wurden Produkte mit endständiger Sulfonsäuregruppe und benachbarter Sulfinatgruppe als wahrscheinlicher, aber unbewiesener Struktur erhalten (DE-PS-1117565).

Des weiteren sind aus J. Amer. Pharm. Assoc. 46 (1957) 578 Propan-1,2,3-trisulfonate als Umsetzungsprodukte von 1,2,3-Trihalogenpropan mit Sulfiten bekannt, die jedoch nur als Nebenprodukte in geringer Ausbeute als Bestandteil eines komplexen Propansulfonsäuregemisches erhalten wurden, wie auch durch Nacharbeiten der Literaturangaben unter Einsatz moderner spektrometrischer Analysenmethoden gezeigt werden kann (s. das Vergleichsbeispiel).

Die Anwendung dieser Verfahrensweise auf Allylsulfonat als Ausgangsprodukt für die Herstellung von reaktiven Propansulfonaten ist bisher nicht beschrieben worden.

Der vorliegenden Erfindung liegt die Aufgabe zugrunde, neue mehrbasige Propansulfonsäuren und deren Salze mit wertvollen Eigenschaften, ein einfaches, auf dem Einsatz preisgünstiger technischer Ausgangsmaterialien beruhendes Verfahren zur formelreinen Herstellung dieser Verbindungen und ihre Verwendung, insbesondere als Hydrotropika, anzugeben.

Diese Aufgabe wird anspruchsgemäss gelöst. Die erfindungsgemässen mehrbasigen Propansulfonsäuren besitzen die Formel I,

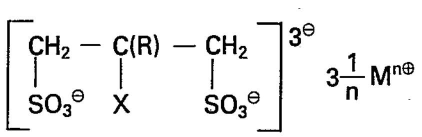

(I),

in der bedeuten:

X $SO_2^{\ominus}$ oder $SO_3^{\ominus}$,

R Wasserstoff oder Methyl,

M gleiche oder verschiedene Kationen, und

n die Wertigkeit des jeweiligen Kations M.

M bedeutet insbesondere Metallkationen, wie $Na^{\oplus}$ oder $K^{\oplus}$, N-haltige Kationen, wie $NH_4^{\oplus}$ oder ein Alkylammoniumion, oder auch $H^{\oplus}$.

M kann ferner auch zwei- oder höherwertige Kationen, insbesondere Metallkationen, bedeuten.

Das erfindungsgemässe Verfahren zur Herstellung der Verbindungen der Formel I ist gekennzeichnet durch Umsetzung eines Alkali- oder Ammoniumallylsulfonats oder eines entsprechenden Metallylsulfonats mit mindestens der einfachen molaren Menge eines Alkali- oder Ammoniumhydrogensulfits in Anwesenheit eines Alkali- oder Ammoniumperoxodisulfats, gegebenenfalls in Kombination mit anderen Oxidationsmitteln, in wässriger Lösung bei Raumtemperatur im pH-Wertbereich von 1,5 bis 4,0.

Wird das Peroxodisulfat in einer Menge von 1 bis 8 Mol-% zugegeben, so werden 1,3-Disulfonato-2-sulfinatopropane bzw. 1,3-Disulfonato-2-sulfinato-2-methylpropane gebildet, welche entweder isoliert oder direkt als Lösung weiterverarbeitet werden können:

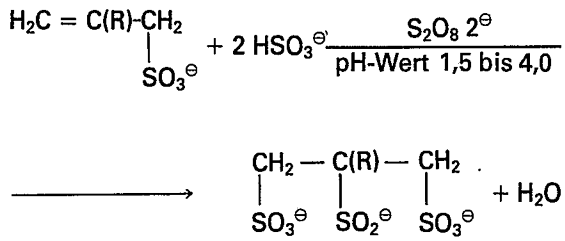


Eine in Anlehnung an den Stand der Technik durchgeführte Umsetzung von Allylsulfonat mit Hydrogensulfit in Gegenwart von Luftsauerstoff im bevorzugten pH-Wertbereich von 4 bis 5 ergab erwartungsgemäss 1,3-Disulfonato-2-sulfinatopropan, allerdings auch gleichzeitig in nicht unerheblichem Masse 1,3-Disulfonatopropan und 1,2,3-Trisulfonatopropan als Nebenprodukte. Zugleich lässt der Einsatz von Peroxiden zur Initiierung radikalischer Sulfitreaktionen nach Angaben aus der DE-OS-2313539 (Seite 4) wenig Erfolg erwarten, da «...die Verwendung von Peroxiden für die freie Radikalinitiierung zu einer schnellen und bevorzugten Reaktion mit Bisulfit führt. Als Folge davon können beträchtliche Mengen an Peroxid bei der Umsetzung mit der Sulfitverbindung verbraucht werden, wodurch die für die freie Radikalinitiierung verfügbare Menge herabgesetzt wird...». So konnte auch bei Einsatz organischer Peroxide oder anderer organischer Radikalbildner kein einheitliches Reaktionsprodukt erzielt werden.

Es war nicht zu erwarten und sehr überraschend, dass bei Verwendung von Peroxodisulfaten als Radikalbildner die Addition von Hydrogensulfiten an Allyl- bzw. Methallylsulfonate in 4- bis 120fach höherer Reaktionsgeschwindigkeit zu reinem 1,3-Disulfonato-2-sulfinatopropan bzw. 1,3-Disulfonato-2-sulfinato-2-methylpropan führt, wenn man gleichzeitig den pH-Wert des Reaktionsgemisches in den Bereich zwischen 1,5 und 4,0 absenkt. Um eine so hohe Reaktionsgeschwindigkeit und Selektivität zu erzielen, kann man den Initiator im Unterschied zu bekannten Initiierungsweisen auf einmal zusetzen.

Erfindungsgemäss erhält man in exothermer Reaktion in Minuten oder sogar Sekunden in quantitativem Umsatz das 1,3-Disulfonato-2-sulfinatopropan bzw. das entsprechende 1,3-Disulfonato-2-sulfinato-2-methylpropan.

Die ausschlaggebende Rolle des Peroxodisulfats als Initiator und der überraschende Reaktionsverlauf ergeben sich daraus, dass auch bei sorgfältigem Ausschluss von Sauerstoff das gleiche Resultat erzielt wird.

Erfahrungsgemäss ist bei Erreichen des Temperaturmaximums die Umsetzung auch beendet. In der Figur ist die Abhängigkeit der Dauer der exothermen Reaktion von der eingesetzten Initiatormenge (Ammoniumperoxodisulfat, $(NH_4)_2S_2O_8$ = APS) bei einem pH-Wert von 2,7 und einer Konzentration von 1 mol Natriumallylsulfonat und 2,1 mol Natriumhydrogensulfit pro kg Reaktionsgemisch (14,4 Masse-% Natriumallylsulfonat) dargestellt. Bei Einsatz von 2 Mol-% APS werden nur 90 sek bis zum vollständigen Umsatz benötigt; bei 1 Mol-% Initiator verlängert sich die Reaktionszeit auf 4 min. Jedoch beträgt bei 8 Mol-% Initiator die Reaktionszeit bis zum vollständigen Umsatz nur noch 15 sek, was aber für das präparative Arbeiten keinen nennenswerten Vorteil bietet. Im Bereich von 0,5 Mol-% APS und weniger wird die Reaktion in der Anfangsphase von der Perverbindung initiiert und kann dann auch durch Sauerstoffeinwirkung weitergeführt werden. Die ausschliessliche Initiierung mit Luftsauerstoff, beispielsweise durch Einrühren in die Reaktionslösung, erfordert längere Reaktionszeiten bis zum Erreichen des Temperaturmaximums (115 min), wobei jedoch der Umsatz nicht vollständig ist. Man kann zwar durch Einleiten von Luftsauerstoff in eine kräftig gerührte Reaktionslösung die Reaktionszeit weiter verkürzen, jedoch werden dabei auch erhebliche Mengen an Schwefeldioxid ausgetragen, was ein Ansteigen des pH-Wertes bzw. eine Verringerung der Sulfinatausbeute zur Folge hat.

Bei höherer Ausgangskonzentration (19,6 Masse-% Natriumallylsulfonat) und 2 Mol-% Kaliumperoxodisulfat als Initiator beträgt die Reaktionszeit bis zum vollständigen Umsatz nur noch 40 sek.

Im pH-Wertbereich von 1,5 bis 4,0 sinkt die Selektivität in bezug auf 1,3-Disulfonato-2-sulfinatopropan von 100% auf 90%; bei höheren pH-Werten sinkt die Selektivität noch stärker durch steigende Bildung von 1,3-Disulfonatopropan; andere stellungsisomere Strukturen wurden mittels $^{13}$-C-NMR-Spektroskopie ausgeschlossen.

Werden das Allylsulfonat oder das Methallylsulfonat erfindungsgemäss anstatt mit einer katalytischen Menge von 1 bis 8 Mol-% mit einem Moläquivalent eines Alkali- oder Ammoniumperoxodisulfats in wässriger Lösung zur Umsetzung gebracht, so entstehen Propan-1,2,3-trisulfonsäure bzw. 2-Methylpropan-1,2,3-trisulfonsäure oder deren Salze:

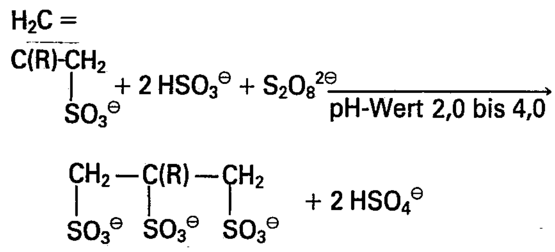


Zweckmässig verfährt man erfindungsgemäss so, dass man das Allylsulfonat – beispielsweise grosstechnisch hergestelltes Natriumallylsulfonat – und das Hydrogensulfit im Molverhältnis 1:2 in Wasser löst, einen pH-Wert von 2,0 einstellt und unter Rühren das Peroxodisulfat einträgt, wobei sich die fortschreitende Reaktion durch rasche Erwärmung des Reaktionsgemisches zu erkennen gibt, das dabei bis zum Sieden kommen kann. Die überraschende Disulfonierung der C=C-Doppelbindung erfolgt rasch und in quantitativer Ausbeute. Die Einheitlichkeit des Umsetzungsproduktes sowie die Vollständigkeit des Umsatzes des eingesetzten Allylsulfonats lassen sich NMR-spektrometrisch einfach nachweisen.

Die Reaktion ist spezifisch an die Anwesenheit von Peroxodisulfat gebunden. Andere Perverbindungen, z. B. Wasserstoffperoxid oder Perborate, lösen keine vergleichbare Reaktion aus, sondern oxidieren lediglich die Sulfite zu Sulfaten. Es ist jedoch möglich, Peroxodisulfat und andere Oxidationsmittel in Kombination einzusetzen, derart, dass dann weniger als 1 Moläquivalent Persulfat benötigt wird, um 1 mol Allylsulfonat in das Propantrisulfonat zu überführen. Als kombinierbare Oxidationsmittel eignen sich insbesondere Wasserstoffperoxid, Chlor, Chlorat oder Bromat.

Infolge Gleichgewichtseinstellung zwischen gebildetem Hydrogensulfat und Propantrisulfonat bzw. 2-Methylpropantrisulfonat liegt im Reaktionsgemisch nach vollständigem Umsatz überwiegend Propan-1,2,3-trisulfonsäure bzw. 2-Methylpropan-1,2,3-trisulfonsäure vor, so dass die erhaltene Reaktionslösung für viele Zwecke ohne weitere Aufarbeitung verwendet werden kann.

Neutralisation der Reaktionslösung mit beliebigen Basen liefert die Propan-1,2,3-trisulfonate oder 2-Methylpropan-1,2,3-trisulfonate. Mit dem erfindungsgemässen Verfahren ist damit die Gewinnung einer formelreinen mehrbasigen Propansulfonsäure möglich, welche bisher noch nicht frei von anderen Propansulfonsäuren erhalten werden konnte (s. hierzu das Vergleichsbeispiel, das dem Stand der Technik nachgearbeitet wurde).

## Ausführungsbeispiele

Die in den nachfolgenden Beispielen aufgeführten $^{13}$-C-NMR-Spektren wurden in $D_2O$ gemessen, als externer Standard diente Tetramethylsilan (TMS). Die Zahlenangaben an den C-Atomsymbolen der Strukturformeln entsprechen den chemischen Verschiebungen in ppm.

### Beispiel 1

a) Trinatriumsalz von 1,3-Disulfonato-2-sulfinatopropan

200 g (1 mol) eines 72%igen technischen Natriumallylsulfonats mit einem Gehalt von 25% NaCl und 3% $Na_2SO_4$ werden unter Rühren in eine Mischung aus 618,5 g (2,1 mol) 35,33%iger technischer Natriumhydrogensulfitlösung ($Fe^{2+}$-Gehalt 8 mg/l) und 150 g Leitungswasser eingetragen.

Nach Einstellung eines pH-Wertes von 2,1 durch Zusatz von 17 g konz. Salzsäure initiiert man die Reaktion durch Zusatz einer Lösung von 4,56 g (2 Mol-%) Ammoniumperoxodisulfat in 10 g Wasser. Beginnend bei 20 °C steigt die Temperatur des Reaktionsgemisches an, erreicht nach 30 sek 50 °C, nach 60 sek 53 °C und nach 90 sek 53,5 °C als Maximum, wonach die Umsetzung beendet ist. Der Umsatz ist quantitativ, wie durch jodometrische Bestimmung des Restsulfits und durch bromatometrische Bestimmung des Sulfinats in salzsaurer Lösung belegt werden kann. Aufgrund des $Fe^{2+}$-Gehaltes der technischen Chemikalien wird sofort nach Initiatorzugabe die rote Farbe des $Fe^{3+}$-Sulfinats beobachtet; diese Färbung kann im fertigen Reaktionsprodukt entweder durch Zusatz entfärbender Komplexbildner, z. B. Dimethylaminomethanbisphosphonsäure, oder durch Einstellen eines pH-Wertes um 7 und Oxidation des $Fe^{2+}$ zu $Fe^{3+}$, welches dann als Eisen(III)-hydroxid ausgefällt und durch Filtration abgetrennt werden kann, beseitigt werden.

Ein von der Reaktionslösung aufgenommenes $^{13}C$-NMR-Spektrum beweist das Vorliegen einer symmetrischen Struktur:

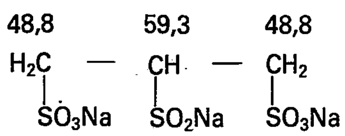

b) Trinatriumsalz von 1,3-Disulfonato-2-sulfinatopropan

Zu einer Lösung von 2582,5 g (10,1 mol) einer 40,7%igen technischen Natriumhydrogensulfitlösung mit einem Eisengehalt von 60 mg/l werden 1000 g (5 mol) 72%iges technisches Natriumallylsulfonat unter Rühren zugefügt. Die erhaltene Suspension wird mit 55 g konz. Salzsäure versetzt, wobei sich der pH-Wert der Lösung auf 2,3 erniedrigt. Man gibt auf einmal 24 g (2 Mol-%) feingepulvertes Natriumperoxodisulfat unter Rühren hinzu, wobei sich die Reaktionslösung sofort blutrot färbt und homogenisiert. In 40 sek erhöht sich die Temperatur der Lösung von 31 auf 70 °C, wonach die Umsetzung quantitativ ist. Lässt man nun die Reaktionslösung unter Rühren auf etwa 50 °C abkühlen, beginnt bereits die Auskristallisation des farblosen Trinatriumsalzes des 1,3-Disulfonato-2-sulfinatopropans. Nach weiterem Abkühlen auf Raumtemperatur kann so in einer Ausbeute von ca. 75% reines Sulfinat abgetrennt werden. Beim Aufbewahren im Kühlschrank lässt sich die Ausbeute an kristallinem Produkt noch erhöhen.

Das Trinatriumsalz lässt sich aus Wasser umkristallisieren.

Beispiel 2

Trinatriumsalz von 1,3-Disulfonato-2-sulfinatopropan neben dem Dinatriumsalz von 1,3-Disulfonatopropan.

Man verfährt wie in Beispiel 1a und 1b beschrieben, jedoch beim pH-Wert 5. Die Einstellung des pH-Wertes erfolgt durch Zugabe von Natronlauge zur Allylsulfonat/Hydrogensulfit-Lösung bzw. -Suspension. Hierbei tritt in verstärktem Masse (ca. 50%) das Dinatriumsalz des 1,3-Disulfonatopropans auf, dessen Bildung NMR-spektroskopisch belegt wurde.

$^{13}C$-NMR-Spektrum:

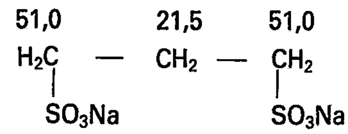

Beispiel 3

Trinatriumsalz von 1,3-Disulfonato-2-sulfinato-2-methylpropan neben dem Trinatriumsalz von 1,2,3-Trisulfonato-2-methylpropan und dem Dinatriumsalz von 1,3-Disulfonato-2-methylpropan

7,9 g (50 mmol) Natriummethallylsulfonat (aus Wasser umkristallisiertes, einheitliches Produkt mit den $^{13}C$-NMR-Signalen bei 138,5 ppm (C); 119,4 ppm ($CH_2$=); 60,4 ppm ($CH_2$-$SO_3^{\ominus}$) und 23,4 ppm (-$CH_3$)) werden unter Rühren in 10,6 g Leitungswasser gelöst, mit 30,9 g (105 mmol) 35,33%iger technischer Natriumhydrogensulfitlösung gemischt (pH-Wert der Mischung 4,0) und auf einmal mit 0,57 g (5 Mol-%) kristallinem Ammoniumperoxodisulfat versetzt. Beginnend bei 23 °C steigt die Temperatur des Reaktionsgemisches (1 mol Natriummethallylsulfonat pro kg der Mischung) an, erreicht nach 40 sek 42 °C und nach 70 sek 50 °C als Maximum, wonach die Umsetzung beendet ist.

Ein von der neutralisierten Reaktionslösung aufgenommenes $^{13}C$-NMR-Spektrum zeigt folgende Produktzusammensetzung:

Hauptprodukt:

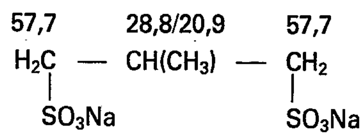

und

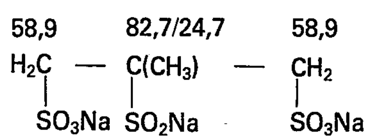

Spuren:

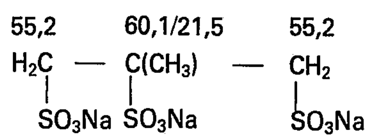

Beispiel 4

Trinatrium-propan-1,2,3-trisulfonat aus Natriumallylsulfonat

In einen mit Rührer, Rückflusskühler, Tropftrichter und Thermometer ausgerüsteten Sulfierkolben werden zunächst 200 g (1 mol) 72%iges technisches Natriumallylsulfonat mit einem Gehalt von 25% NaCl und 3% $Na_2SO_4$, 200 g Wasser und 35 g 37%ige Salzsäure miteinander vermischt, wobei sich der grösste Teil des Allylsulfonats auflöst.

Dann fügt man 533,6 g (2 mol) 39%ige technische wässrige Natriumhydrogensulfitlösung mit einem Eisengehalt von 9 mg/mol Lösung hinzu und erhält eine homogene, fahlgelbe Lösung, deren pH-Wert 2,0 (Glaselektrode) beträgt. Zu der so vorbereiteten Startlösung dosiert man eine 40%ige wässrige Natriumperoxodisulfatlösung, die aus 238,1 g (1 mol) Natriumperoxodisulfat und 357,15 g Wasser bereitet wird, in dem Masse hinzu, dass sich die reagierende Lösung in etwa 2,5 min, beginnend bei Raumtemperatur, zum Sieden erwärmt. Man setzt die Zugabe der Peroxodisulfatlösung so zügig fort, dass die Reaktionswärme leicht durch Siedekühlung abgeführt werden kann, wofür nochmals 1,5 min benötigt werden. Zu Beginn der Peroxodisulfatzugabe färbt sich die reagierende Lösung unter zunehmender Erwärmung blutrot, hellt sich dann mit fortschreitender Zugabe allmählich auf und ist, nachdem etwa 80% zudosiert sind, fast farblos und zum Schluss fahlgrün bis gelb.

Die folgende Übersicht zeigt den zeitlichen Verlauf der exothermen Reaktion während der Zudosierphase des Oxidationsmittels:

| Zeit (s) | 0 | 45 | 75 | 120 | 150 | 180 | 240 |
|---|---|---|---|---|---|---|---|
| Temp.(°C) | 22 | 40 | 60 | 80 | 96 | 104 | 104 |

Nach Neutralisation der stark sauren Reaktionslösung mit 33%iger Natronlauge flockt das durch Einsatz technischer Chemikalien eingebrachte Eisen als Eisen(III)-hydroxid aus und kann zusammen mit dem grössten Teil des Natriumsulfathydrats abfiltriert werden.

Ein vom farblosen Filtrat aufgenommenes $^1$H- bzw. $^{13}$C-NMR-Spektrum bestätigt die quantitative und selektive Umwandlung des Allylsulfonats in das Propantrisulfonat.

$^{13}$C-NMR-Spektrum:

52,5 55,2 52,5

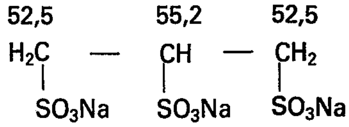

Beispiel 5

Trinatrium-propan-1,2,3-trisulfonat

Dieses Beispiel soll die Eignung von Oxidationsmittelkombinationen veranschaulichen.

Nach Beispiel 4 wird eine homogene Startlösung vom pH-Wert 2 hergestellt und auf einmal mit 11,9 g (5 Mol-%) feingepulvertem Natriumperoxodisulfat versetzt. Das Peroxodisulfat löst sich schnell auf, und die reagierende Lösung erwärmt sich innerhalb von 2 min von Raumtemperatur auf 56 °C. Nun dosiert man im Verlauf von 3 min 107,7 g (95 Mol-%) 30%iges Wasserstoffperoxid hinzu, wobei sich die Lösung weiter bis zum Sieden erwärmt und die Reaktionswärme durch Siedekühlung abgeführt wird. Zum Schluss der Peroxidzugabe ist die Reaktionslösung praktisch farblos und siedet bei 105 °C.

Die folgende Übersicht zeigt den zeitlichen Verlauf der exothermen Reaktion während der Zudosierungsphase der Oxidationsmittel.

| Zeit (min) | 0 | 1 | 2 | 2,5 | 3 | 3,5 | 5 |
|---|---|---|---|---|---|---|---|
| Temp.(°C) | 22 | 52 | 56 | 65 | 90 | 104 | 105 |

Nach der Neutralisation der sauren Reaktionslösung mit 86 g 33%iger Natronlauge flockt wenig Eisen(III)-hydroxid aus.

Die NMR-Spektren des farblosen Filtrats zeigen, dass die erhaltene Reaktionslösung reines Propantrisulfonat enthält. Durch Einengen der wässrigen Lösung zur Trockne kann das Trisulfonat neben den anorganischen Salzen kristallin erhalten werden. Eine Abtrennung von den Begleitsalzen gelingt durch Umkristallisation aus Wasser. Durch Kationenaustausch lässt sich die freie Propan-1,2,3-trisulfonsäure als fast farbloses, viskoses Öl erhalten.

Beispiel 6

Trinatrium-2-methylpropan-1,2,3-trisulfonat neben Dinatrium-2-methylpropan-1,3-disulfonat

Aus 7,9 g (50 mmol) Natriummethallylsulfonat (aus Wasser umkristallisiertes, einheitliches Produkt mit den $^{13}$C-NMR-Signalen bei 138,5 ppm (C); 119,4 ppm ($CH_2$=); 60,4 ppm ($CH_2 \cdot SO_3^{\ominus}$) und 23,4 ppm ($-CH_3$)), 10,6 g Leitungswasser und 30,9 g (105 mmol) 35,33%iger technischer Natriumhydrogensulfitlösung wird eine homogene Mischung vom pH-Wert 4 hergestellt und nach Beispiel 5 mit 5 Mol-% Natriumperoxodisulfat und 95 Mol-% 30%igem Wasserstoffperoxid umgesetzt. Ein von der neutralisierten Reaktionslösung angefertigtes $^{13}$C-NMR-Spektrum zeigt folgende Produktzusammensetzung:

Hauptprodukt

57,7 28,8/20,5 57,7

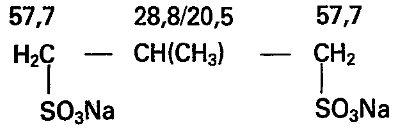

55,3 60,1/21,4 55,3

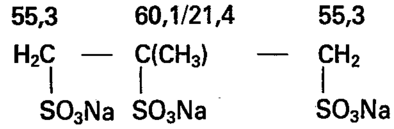

Beispiel 7

Trinatrium-propan-1,2,3-trisulfonat neben Dinatriumpropan-1,3-disulfonat

Dieses Beispiel soll die Tatsache demonstrieren, dass die Selektivität der ablaufenden Reaktion abnimmt, also keine einheitlichen Reaktionsprodukte erhalten werden, wenn der pH-Wert der homogenen Startlösung auf Werte >2 eingestellt wird.

Man löst 1 mol 72%iges technisches Natriumallylsulfonat in 2 mol 39%iger Natriumhydrogensulfitlösung auf. Setzt man nun diese Mischung, deren pH-Wert 4,8 (Glaselektrode) beträgt, nach der in Beispiel 4 oder 5 beschriebenen Arbeitsweise mit einem Peroxodisulfat oder einer Peroxodisulfat/Wasserstoffperoxid-Kombination um, so werden neben Propan-1,2,3-trisulfonat beträchtliche Mengen Propan-1,3-disulfonat erhalten, wie $^{1}H$- bzw. $^{13}C$-NMR-spektrometrisch nachgewiesen werden konnte.

Wählt man jedoch eine Arbeitsweise, die es gestattet, Reaktionsbedingungen bei pH-Werten $\geq 7$ zu realisieren, wie dies beispielsweise in der DD-PS-154443 beschrieben ist, gelingt es, Natriumallylsulfonat selektiv und quantitativ in Propan-1,3-disulfonat umzuwandeln.

$^{13}C$-NMR-Spektrum des Dinatrium-propan-1,3-disulfonats:

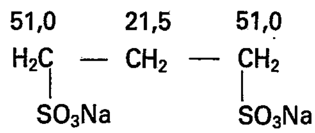


Vergleichsbeispiel

Trinatrium- propan-1,2,3-trisulfonat aus 1,2,3-Tribrompropan

Das $^{13}C$-NMR-Spektrum eines aus 1,2,3-Tribrompropan und Natriumsulfit nach W.D. Roll und G.E. Cwalina, J. Amer. Pharm. Assoc. 4 (1957) 578, hergestellten Umsetzungsprodukts zeigt, dass es sich nicht um ein einheitliches Produkt, sondern um ein Gemisch mehrerer Propansulfonate handelt, von denen das Trinatrium-propan-1,2,3-trisulfonat nur ein untergeordneter Bestandteil ist. Das für den Versuch verwendete 1,2,3-Tribrompropan war ein einheitliches Produkt mit $^{13}C$-NMR-Signalen bei 48,3 ppm und 35,0 ppm.

$^{13}C$-NMR-Spektren des erhaltenen Produktgemisches in ppm:

137,6; 130,9; 59,2; 55,2; 54,4; 52,7; 51,2; 41,2; 21,8.

Neben Propan-1,2,3-trisulfonat (55,2; 52,7) und weiteren, nicht identifizierten Bestandteilen, konnten Propan-1,3-disulfonat (51,2; 21,8) und Propen-1,3-disulfonat (137,6; 130,9; 59,2) erkannt werden.

**Patentansprüche**

1. Mehrbasige Propansulfonsäuren der Formel I,

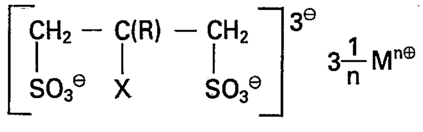
(I),

in der bedeuten:

X $SO_2^{\ominus}$ oder $SO_3^{\ominus}$,
R Wasserstoff oder Methyl,
M gleiche oder verschiedene Kationen und
n die Wertigkeit der Kationen.

2. Mehrbasige Propansulfonsäuren der Formel I nach Anspruch 1, in der M ein einwertiges Kation (n = 1) bedeutet.

3. Mehrbasige Propansulfonsäuren der Formel I nach Anspruch 1 oder 2, in der M ein einwertiges N-haltiges Kation (n = 1) bedeutet.

4. Mehrbasige Propansulfonsäuren der Formel I nach Anspruch 3, in der M $NH_4^{\oplus}$ oder ein Alkylammoniumion bedeutet.

5. Mehrbasige Propansulfonsäuren der Formel I nach Anspruch 1 oder 2, in der M $H^{\oplus}$ oder Metallkationen, wie $Na^{\oplus}$ oder $K^{\oplus}$, bedeutet.

6. Verfahren zur Herstellung der mehrbasigen Propansulfonsäuren der Formel I nach einem der Ansprüche 1 bis 5, gekennzeichnet durch Umsetzung von Alkali- oder Ammoniumallylsulfonaten oder entsprechenden Methallylsulfonaten der Formel II,

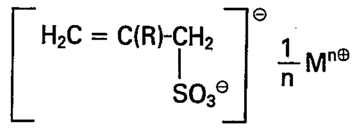
(II)

mit R, m und n wie in Anspruch 1

mit der mindestens zweifachen molaren Menge eines Alkali- oder Ammoniumhydrogensulfits in Anwesenheit eines Alkali- oder Ammoniumperoxodisulfats, gegebenenfalls in Kombination mit anderen Oxidationsmitteln, in wässriger Lösung bei Raumtemperatur im pH-Wertbereich von 1,5 bis 4,0.

7. Verfahren nach Anspruch 6, dadurch gekennzeichnet, dass das Alkali- oder Ammoniumperoxodisulfat in katalytischer Menge von 1 bis 8 Mol-%, bezogen auf das eingesetzte Allylsulfonat oder Methallylsulfonat, zugegeben wird.

8. Verfahren nach Anspruch 6, dadurch gekennzeichnet, dass das Alkali- oder Ammoniumperoxodisulfat allein in einer Menge von einem Moläquivalent oder in Kombination mit anderen Oxidationsmitteln unter Verfügbarkeit von zwei Oxidationsäquivalenten, bezogen auf das eingesetzte Allylsulfonat oder Methallylsulfonat, zugegeben wird.

9. Verfahren nach Anspruch 6 oder 8, dadurch gekennzeichnet, dass als in Kombination mit Peroxodisulfat eingesetzte Oxidationsmittel Chlor oder Chlor abgebende Stoffe, Chlorate, Bromate oder Wasserstoffperoxid eingesetzt werden.

10. Hydrotrope Zusammensetzungen, gekennzeichnet durch mindestens eine mehrbasige Propansulfonsäure der Formel I nach einem der Ansprüche 1 bis 5.

**Claims**

1. Polybasic propane sulphonic acids of the formula I,

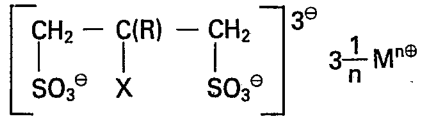
(I),

in which signify:
X $SO_2^-$ or $SO_3^-$,
R hydrogen or methyl radical,
M equal or different cations and
n valency of the cations.

2. Polybasic propane sulphonic acids of the formula I according to claim 1, characterized in that M denote a monovalent cation (n = 1).

3. Polybasic propane sulphonic acids of the formula I according to claim 1 or 2, characterized in that M denote a monovalent N-containing cation (n = 1).

4. Polybasic propane sulphonic acids of the formula I according to claim 3, characterized in that M denote $NH_4^+$ or alkyl ammonium ion.

5. Polybasic propane sulphonic acids of the formula I according to claim 1 or 2, characterized in that M denote $H^+$ or metallic cations, like $Na^+$ or $K^+$.

6. Process for the preparation of the polybasic propane sulphonic acids of the formula I according to any of claims 1 to 5, characterized by reaction of an alkali or ammonium allyl sulphonate or the corresponding methallyl sulphonate of the formula II,

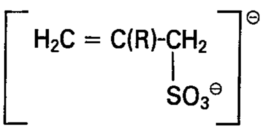 $\frac{1}{n}M^{n\oplus}$ (II)

wherein R, M and n the same as in claim 1, with at least the twofold molar amount of a alkali or ammonium hydrogen sulphite in the presence of an alkali or ammonium peroxodisulphate, possibly in combination with other oxidation agents, in aqueous solution at room temperature and at a pH-value in the range of 1,5 to 4,0.

7. Process according to claim 6, characterized in that the alkali or ammonium peroxodisulphate is added in catalytic amounts between 1 to 8 Mol-%, calculated with respect to the allyl sulphonate or methallyl sulphonate used.

8. Process according to claim 6, characterized in that the alkali or ammonium peroxodisulphate is added alone in a molar amount or in combination with other oxidation agents with the availability of 2 oxidation equivalents, referred to allyl sulphonate or methallyl sulphonate used.

9. Process according to claim 6 or 8, characterized in that in combination with peroxodisulphate, as oxidation agents there are used chlorine or materials giving off chlorine, chlorates, bromates or hydrogen peroxide.

10. Hydrotropic compounds characterized by at least a polybasic propane sulphonic acid of the formula I according to any of claims 1 to 5.

**Revendications**

1. Acides propane-sulfoniques polybasiques de formule I:

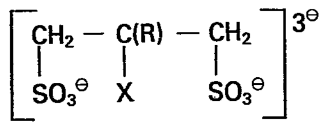 $3\frac{1}{n}M^{n\oplus}$ (I),

dans laquelle
X signifie $SO_2^{\ominus}$ ou $SO_3^{\ominus}$,
R de l'hydrogène ou méthyle,
M des cations identiques ou différents et
n la valence des cations.

2. Acides propane-sulfoniques polybasiques de formule I selon la revendication 1, dans laquelle M est un cation monovalent (n = 1).

3. Acides propane-sulfoniques polybasiques selon la revendication 1 ou 2, dans laquelle M signifie un cation renfermant N monovalent (n = 1).

4. Acides propane-sulfoniques polybasiques de formule I selon la revendication 3, dans laquelle M signifie $NH_4^{\oplus}$ ou un ion alcoyl-ammonium.

5. Acides propane-sulfoniques polybasiques de formule I selon la revendication 1 ou 2, dans laquelle M signifie $H^{\oplus}$ ou des cations métalliques tels que $Na^{\oplus}$ ou $K^{\oplus}$.

6. Procédé de préparation des acides propane-sulfoniques polybasiques de formule I selon l'une des revendications 1 à 5, caractérisé par la réaction d'allylsulfonates alcalins ou d'ammonium ou des méthallylsulfonates correspondants de formule II

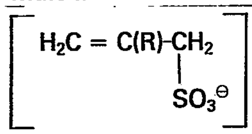 $\frac{1}{n}M^{n\oplus}$ (II)

avec R, M et n comme à la revendication 1, avec au moins deux fois la quantité molaire d'un hydrogénosulfite alcalin ou d'ammonium en présence d'un peroxodisulfate alcalin ou d'ammonium, éventuellement en combinaison avec d'autres agents oxydants, en solution aqueuse à la température ordinaire, dans la gamme de valeurs de pH de 1,5 à 4,0.

7. Procédé selon la revendication 6, caractérisé en ce qu'on ajoute le peroxodisulfate alcalin ou d'ammonium en une quantité catalytique de 1 à 8 Mol-% par rapport à l'allylsulfonate ou méthallylsulfonate employé.

8. Procédé selon la revendication 6, caractérisé en ce que le peroxodisulfate alcalin ou d'ammonium est ajouté à lui seul en une quantité d'un équivalent molaire ou, en combinaison avec d'autres agents oxydants, tout en disposant de deux équivalents d'oxydation, par rapport à l'allylsulfonate ou méthallylsulfonate employé.

9. Procédé selon la revendication 6 ou 8, caractérisé en ce qu'on utilise comme agents oxydants, employés en combinaison avec le peroxodisulfate, du chlore ou des substances libérant du chlore, des chlorates, des bromates ou du peroxyde d'hydrogène.

10. Compositions hydrotropes, caractérisées par au moins un acide propanesulfonique polybasique de formule I selon l'une des revendications 1 à 5.

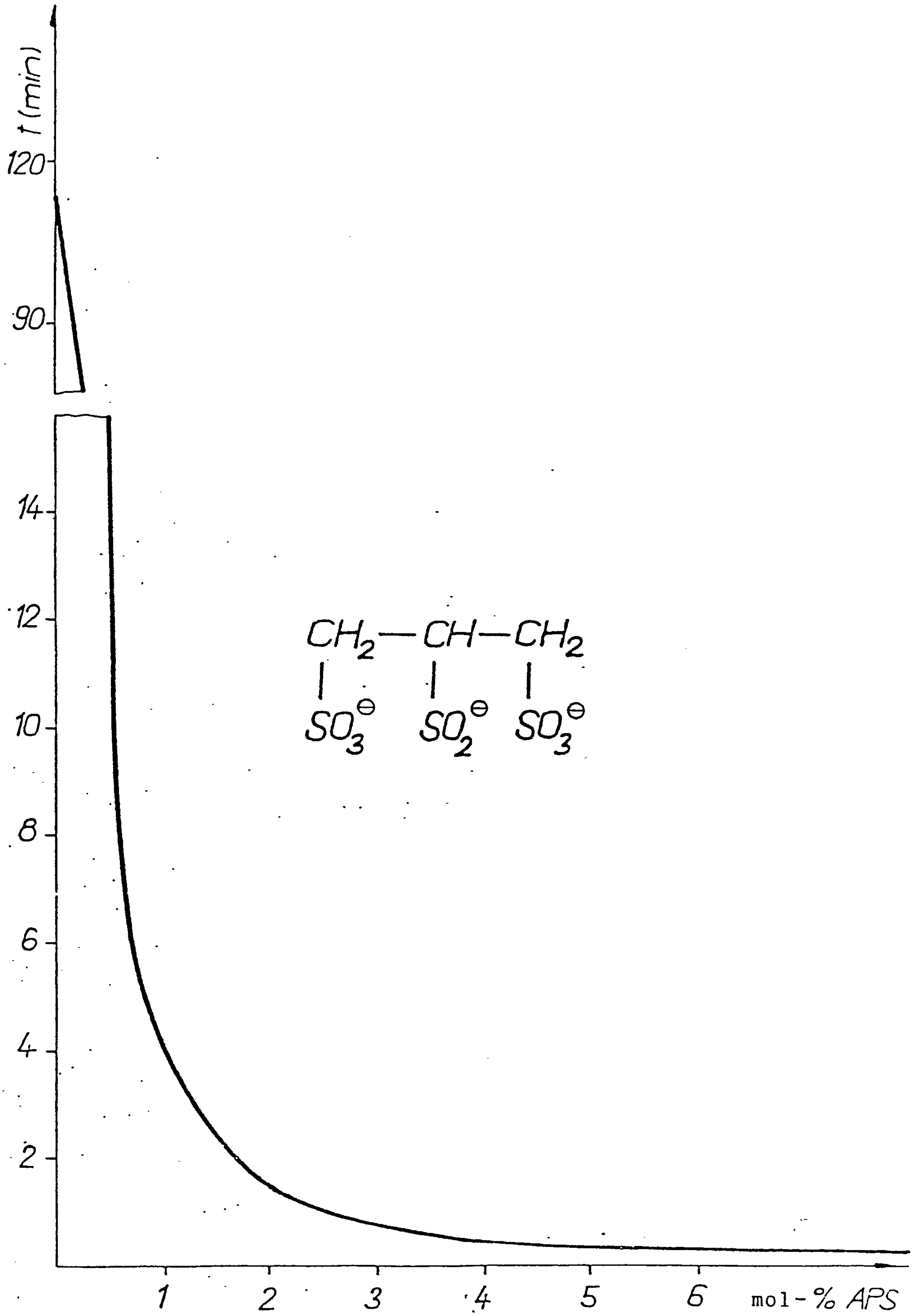
t (min)
120
90
14
12
10
8
6
4
2
1
2
3
4
5
6
mol-% APS